# EUROPEAN PATENT APPLICATION

(11) **EP 2 077 335 A1**
(43) Date of publication of application: **08.07.2009**
(21) Application number: 07025080.8
(22) Date of filing: 22.12.2007
(51) Int. Cl.: C12Q 1/68

(54) **EXO1 promoter polymorphism associated with exceptional life expectancy in humans**

(71) Applicant: Universitätsklinikum Schleswig-Holstein, 24105 Kiel (DE)
(72) Inventor: Schreiber, Stefan, Prof. Dr., 24105 Kiel (DE); Nebel, Almut, Dr., 24105 Kiel (DE); Till, Andreas, Dr., 24105 Kiel (DE); Flachsbart, Friederike, 24114 Kiel (DE); Rosenstiel, Philip, 24116 Kiel (DE)
(74) Representative: Biehl, Christian

(57) **Abstract**

The invention relates to an exonuclease 1 (*EXO1*) promoter polymorphism associated with exceptional life expectancy in humans. More specifically, the invention relates to the promoter region of EXO1 where the gene has been found to be mutated. Moreover, the invention relates to a gene regulation mechanism involving transcription factor E47 as a transcriptional repressor of EXO1.

SEQUENCE LISTING

<110>  Universitätsklinikum Schleswig-Holstein

<120>  EXO1 promoter polymorphism associated with exceptional life
       expectancy in humans

<130>  P 5582

<160>  50

<170>  PatentIn version 3.3

<210>  1
<211>  50
<212>  DNA
<213>  Homo sapiens

<400>  1
cgggccggca gcagcggctg cagtcgtatg gcagtgagcc gctgtctacc                50


<210>  2
<211>  50
<212>  DNA
<213>  Homo sapiens

<400>  2
cgggccggca gcagcggctg gagtcgtatg gcagtgagcc gctgtctacc                50


<210>  3
<211>  25
<212>  DNA
<213>  artificial sequence

<220>
<223>  EXO1_p_F (forward primer)

<400>  3
ctaaagtctt tgtctctaag gccca                                           25


<210>  4
<211>  21
<212>  DNA
<213>  artificial sequence

<220>
<223>  EXO1_p_R (reverse primer)

<400>  4
cttccaactc atagggttgc g                                               21


<210>  5
<211>  18
<212>  DNA
<213>  artificial sequence

<220>
<223>  EXO1_p_e01_F (forward primer)

<400>  5
catcagcgcc agtgccac                                                    18


<210>  6
<211>  25
<212>  DNA
<213>  artificial sequence

<220>
<223>  EXO1_p_e01_R (reverse primer)

<400>  6
aacacatact ccactcaggc taagg                                            25


<210>  7
<211>  25
<212>  DNA
<213>  artificial sequence

<220>
<223>  EXO1_e02_F (forward primer)

<400>  7
ttcagtgaac ggagggagat aagag                                            25


<210>  8
<211>  29
<212>  DNA
<213>  artificial sequence

<220>
<223>  EXO1_e02_R (reverse primer)

<400>  8
gttctcaaat ttcagaggtt cttagactc                                        29


<210>  9
<211>  19
<212>  DNA
<213>  artificial sequence

<220>
<223>  EXO1_e02a_F (forward primer)

<400>  9
tgcctggccc agaagaaac                                                   19


<210>  10
<211>  22
<212>  DNA
<213>  artificial sequence

<220>
<223>  EXO1_e02a_R (reverse primer)

<400>  10
agtcagtaat gggcactgag ca                                              22


<210>  11
<211>  24
<212>  DNA
<213>  artificial sequence

<220>
<223>  EXO1_e03_F (forward primer)

<400>  11
attggactcc aagctttctc ttca                                            24


<210>  12
<211>  21
<212>  DNA
<213>  artificial sequence

<220>
<223>  EXO1_e03_R (reverse primer)

<400>  12
ggaagtcatc cctgatttgg g                                               21


<210>  13
<211>  24
<212>  DNA
<213>  artificial sequence

<220>
<223>  EXO1_e04_F (forward primer)

<400>  13
caattcctat gaaaacatct gctt                                            24


<210>  14
<211>  32
<212>  DNA
<213>  artificial sequence

<220>
<223>  EXO1_e04_R (reverse primer)

<400>  14
ttaaaaattg gtatcatata gactacagtg tc                                   32


<210>  15
<211>  24
<212>  DNA
<213>  artificial sequence

<220>
<223>  EX01_e05_F (forward primer)

<400>  15
aagcctaaag catctgggtt aatg                                              24


<210>  16
<211>  26
<212>  DNA
<213>  artificial sequence

<220>
<223>  EX01_e05_R (reverse primer)

<400>  16
gagagagcct tacttatgcc ttagga                                            26


<210>  17
<211>  25
<212>  DNA
<213>  artificial sequence

<220>
<223>  EX01_e06_F (forward primer)

<400>  17
tcctctggct gtgtagcagt atttc                                             25


<210>  18
<211>  24
<212>  DNA
<213>  artificial sequence

<220>
<223>  EX01_e06_R (reverse primer)

<400>  18
cattacacaa acatcaagca gcaa                                              24


<210>  19
<211>  25
<212>  DNA
<213>  artificial sequence

<220>
<223>  EX01_e07_F (forward primer)

<400>  19
ataaagagat gcaaaaagag cttgc                                             25


<210>  20
<211>  25
<212>  DNA
<213>  artificial sequence

<220>
<223>  EXO1_e07_R (reverse primer)

<400>  20
agttctagtg cctcagtcat ttgct                                          25


<210>  21
<211>  28
<212>  DNA
<213>  artificial sequence

<220>
<223>  EXO1_e08_F (forward primer)

<400>  21
gaattttaag tgatgtaaca tggttgtg                                       28


<210>  22
<211>  27
<212>  DNA
<213>  artificial sequence

<220>
<223>  EXO1_e08_R (reverse primer)

<400>  22
ctaagtaatc aggatgcata gtggatg                                        27


<210>  23
<211>  23
<212>  DNA
<213>  artificial sequence

<220>
<223>  EXO1_e09_F (forward primer)

<400>  23
aagacaccaa ctcctgcaga gac                                            23


<210>  24
<211>  25
<212>  DNA
<213>  artificial sequence

<220>
<223>  EXO1_e09_R (reverse primer)

<400>  24
cagagcctgg atatagaaac gctta                                          25


<210>  25
<211>  25
<212>  DNA
<213>  artificial sequence

<220>
<223>  EXO1_e10_F (forward primer)

<400>  25
acagctgttt catcaacagc tattg                                          25


<210>  26
<211>  24
<212>  DNA
<213>  artificial sequence

<220>
<223>  EXO1_e10_R (reverse primer)

<400>  26
ggtatgtgtt tatcagcagg gtga                                           24


<210>  27
<211>  26
<212>  DNA
<213>  artificial sequence

<220>
<223>  EXO1_e11_F (forward primer)

<400>  27
aagtccttat ttttaggaca atggga                                         26


<210>  28
<211>  25
<212>  DNA
<213>  artificial sequence

<220>
<223>  EXO1_e11_R (reverse primer)

<400>  28
tatacccatg taacaaaact gcacg                                          25


<210>  29
<211>  25
<212>  DNA
<213>  artificial sequence

<220>
<223>  EXO1_e12_1_F (forward primer)

<400>  29
aattctaaat gtgtgaatgt tggca                                          25


<210>  30
<211>  24
<212>  DNA
<213>  artificial sequence

<220>
<223>  EXO1_e12_1_R (reverse primer)

<400>  30
gcgttcttga aaaatctcca agac                                              24


<210>  31
<211>  22
<212>  DNA
<213>  artificial sequence

<220>
<223>  EXO1_e12_2_F (forward primer)

<400>  31
tggttgacac agatgtagca cg                                                22


<210>  32
<211>  28
<212>  DNA
<213>  artificial sequence

<220>
<223>  EXO1_e12_2_R (reverse primer)

<400>  32
tgtgaacttt ctgcacagta gagtttag                                          28


<210>  33
<211>  26
<212>  DNA
<213>  artificial sequence

<220>
<223>  EXO1_e13_F (forward primer)

<400>  33
cttcttcaat gaaattggca aatatc                                            26


<210>  34
<211>  27
<212>  DNA
<213>  artificial sequence

<220>
<223>  EXO1_e13_R (reverse primer)

<400>  34
acactgcaag agaaaataag tttcaca                                           27


<210>  35
<211>  18
<212>  DNA
<213>  artificial sequence

<220>
<223> EXO1_e14_F (forward primer)

<400> 35
gccgagatcg caccactg 18


<210> 36
<211> 28
<212> DNA
<213> artificial sequence

<220>
<223> EXO1_e14_R (reverse primer)

<400> 36
aacaaaaact catacgaaac taggaaga 28


<210> 37
<211> 23
<212> DNA
<213> artificial sequence

<220>
<223> EXO1_e15_F (forward primer)

<400> 37
attgggagtg ctcacaggag aac 23


<210> 38
<211> 24
<212> DNA
<213> artificial sequence

<220>
<223> EXO1_e15_R (reverse primer)

<400> 38
aactccagag gattttccca atta 24


<210> 39
<211> 19
<212> DNA
<213> artificial sequence

<220>
<223> E47_si1

<400> 39
tgaaccagcc gcagaggat 19


<210> 40
<211> 19
<212> DNA
<213> artificial sequence

<220>
<223>  E47_si2

<400>  40
gaaggtccgg aaggtcccg                                                    19


<210>  41
<211>  19
<212>  DNA
<213>  artificial sequence

<220>
<223>  E47_si3

<400>  41
ggtgtcaggt gtggttgga                                                    19


<210>  42
<211>  19
<212>  DNA
<213>  artificial sequence

<220>
<223>  E47_si4

<400>  42
gcccacaacc ccgccgggc                                                    19


<210>  43
<211>  22
<212>  DNA
<213>  artificial sequence


<220>
<223>  Sc_TDH1-225F (forward primer)

<400>  43
ccaagaaaga gacccagcta ac                                                22


<210>  44
<211>  22
<212>  DNA
<213>  artificial sequence

<220>
<223>  Sc_TDH1-855R (reverse primer)

<400>  44
caagaaatca gaggagacaa cg                                                22


<210>  45
<211>  19
<212>  DNA

<213>  artificial sequence

<220>
<223>  EXO1-996F (forward primer)

<400>  45
gatgccccac aattgaagg                                                    19


<210>  46
<211>  21
<212>  DNA
<213>  artificial sequence

<220>
<223>  EXO1-1510R (reverse primer)

<400>  46
cgtgctacat ctgtgtcaac c                                                 21


<210>  47
<211>  22
<212>  DNA
<213>  artificial sequence

<220>
<223>  TCF3-266F (forward primer)

<400>  47
cctctcttca tccacattcc tg                                                22


<210>  48
<211>  22
<212>  DNA
<213>  artificial sequence

<220>
<223>  TCF3-1893R (reverse primer)

<400>  48
ccacacctga caccttttcc tc                                                22


<210>  49
<211>  26
<212>  DNA
<213>  artificial sequence

<220>
<223>  GAPDH_F (forward primer)

<400>  49
tgaaggtcgg agtcaacgga tttggt                                            26


<210>  50
<211>  24
<212>  DNA

<213>  artificial sequence

<220>
<223>  GAPDH_R (reverse primer)

<400>  50
catgtgggcc atgaggtcca ccac                                              24

## Description

### FIELD OF THE INVENTION

The invention relates to the gene of exonuclease 1 (EXO1) which has been found to be associated with exceptional life expectancy in humans. More specifically, the invention relates to the promoter region of EXO1 where the gene has been found to be mutated. Moreover, the invention relates to a gene regulation mechanism involving transcription factor E47 as a transcriptional repressor of EXO1.

### BACKGROUND OF THE INVENTION

There is increasing evidence that somatic mutations progressively accumulate with age in various species, including humans (Vijg 2000; Hasty et al. 2003). A wide range of exogenous agents (e.g. UV light) and by-products of cellular metabolism (e.g. reactive oxygen species) are known to harm genetic material. In addition, mutations can arise from spontaneous replication errors (Hasty 2005). Since DNA damage may result in dysregulated cell functions, apoptosis, oncogenic transformation, genomic instability and senescence, maintenance systems are critical for both cellular and organismal homeostasis and survival (Lombard et al. 2005). Therefore, numerous genetically determined pathways that either prevent or resolve DNA damage have evolved as highly conserved "longevity assurance mechanisms" (Hasty 2005). Hart & Setlow suggested already in 1974 that the intrinsic fidelity and activity of the repair machinery in different species may influence their rate of age-related functional decline. Another line of evidence that links DNA repair with aging is that many of the human segmental progeroid disorders, such as Werner Syndrome, are caused by mutations in genes involved in DNA maintenance (reviewed in Hasty et al. 2003). Similarly, studies of mouse models show that genetic defects affecting genomic integrity underlie several of the accelerated aging phenotypes (Hasty et al. 2003; Lombard et al. 2005).

These findings support the notion that variation in genes regulating the levels of somatic maintenance and repair functions could also modulate the normal aging process and determine the periods of "assured longevity" (Kirkwood 2005). In humans it is conceivable that limitations in these survival mechanisms - due to genetic predisposition and/or the influence of environmental factors - could result in frailty, age-related functional impairment and a lower life expectancy. Conversely, the more efficient these functions are, the more likely is an individual to attain old age in good physical and cognitive condition, leading not only to an increase in his life span but also in his 'health span'. In fact, it has been postulated that long-lived individuals (LLI; i.e. nonagenarians and centenarians), who are often characterized by a slowing down of the aging process and a delayed onset in age-related diseases (Hitt et al. 1999), are enriched for favourable variants in "longevity assurance genes" compared to younger controls (Perls et al. 2002). A recent epidemiological study strengthens this hypothesis by showing that the genetic contribution to human life expectancy (∼25 to 32%) is minimal before the age of 60, but becomes particularly important for survival at very advanced ages (Hjelmborg et al. 2006).

### DESCRIPTION OF THE INVENTION

The inventors of the present invention have identified exonuclease 1 (*EXO1*) as a novel susceptibility gene for exceptional life expectancy in humans. EXO1 is involved in various DNA metabolic pathways that affect genomic stability. The present detailed functional and genetic analyses revealed a common allele (C) of the SNP rs1776180 in the *EXO1* promoter that is significantly enriched in 381 German centenarians compared to 409 younger controls. This finding replicates in an independent sample of 559 French centenarians and controls. The C allele leads to the loss of a binding site for the transcription factor E47, resulting in a higher *EXO1* expression. Thus, the investors detected a hitherto undescribed role for E47 as a negative regulator of *EXO1* transcription. Given the observed survival advantage that is associated with the C allele of SNP rs 1776180, *EXO1* can be considered a novel longevity enabling gene. As both E47 and EXO1 are critical for the development of lymphoid cell lineages, it is hypothesized that the beneficial effect of an enhanced EXO1 activity could lie in the mitigation of immune defects that arise during aging. In addition the observed effect might be critical in all tissues that show concomitant expression of EXO1 and E47.

In the present study, the inventors of the present invention analyzed a total of 92 coding SNPs (cSNPs) in 49 DNA repair genes in an extensive sample set of 381 German centenarians and 409 younger controls (60 -75 years old) and identified a novel longevity susceptibility locus, the gene encoding exonuclease 1 (EXO1). Detailed functional and genetic analyses revealed a common allelic variant in the promoter of EXO1 that is enriched in centenarians and that influences gene expression via differential binding of the transcription factor E47.

Therefore, it is an object of the invention to provide a method of identifying individuals carrying a genetic variant correlated to longevity or age related diseases or longevity related traits (e.g. cancer), wherein rs1776180 is amplified with one or more oligonucleotide primers. Another object of the invention is to provide a method of identifying a mutation leading to increased life expectancy. Further, it is an object of the invention to provide a method for preparing a pharmaceutical preparation for the therapy of diseases, e.g. by supporting an individual's immune system or germ cells as well as preventing accumulation of DNA damage leading for instance to cancer or general functional decline. Finally, it is an object of the invention of providing a method of identifying E47-dependent gene regulation networks having an impact on exceptional life expectancy.

A person skilled in the art of genetic susceptibility testing will find the present invention particularly useful for:
- identifying individuals having an EXO1 gene with no mutations, who therefore cannot be said to have an increased life expectancy based on the EXO1 gene;
- identifying individuals having an EXO1 gene with a mutation associated with an increased life expectancy based on the EXO1 gene;
- identifying substances having an impact on the expression of the EXO1 gene;
- preparing pharmaceutical compositions for the treatment of infectious and/or malignant diseases and/or diseases related to aging; and
- performing gene therapy.

### BRIEF DESCRIPTION OF THE FIGURES

Fig. 1 is a schematic representation of the *EXO1* gene structure and the positions of the analyzed SNPs.
Fig. 2 illustrates that coding SNPs do not influence DNA repair capacity of EXO1 after UV B treatment.
Fig. 3 depicts that the C allele of *EXO1* SNP rs1776180C contributes to increased promoter activity.
Fig. 4 shows that transcription factor E47 acts as transcriptional repressor of *EXO1*.
Fig. 5 illustrates the analysis of *EXO1* 5'UTR haplotypes.

### DETAILED DESCRIPTION OF THE INVENTION

### Screen of DNA repair genes reveals EXO1 as a candidate gene

The inventors of the present invention tested 92 cSNPs in 49 selected DNA repair genes for association with the longevity phenotype (cf. Table 3). The screening presents the explorative stage of this experiment that served to identify convincing markers for further functional and genetic follow-up. For the association analyses the most commonly used study design in human genetic longevity research was applied by comparing centenarians (n = 381; aged 100 to 110 years) to younger and specifically matched controls (n = 409; aged 60 to 75 years). The validity and efficacy of this approach have previously been demonstrated (Nebel et al. 2005). All analyzed cSNPs had a minor allele frequency of at least 1%, a minimal overall call rate of 95% and showed no significant departure from Hardy-Weinberg equilibrium (HWE) in controls (*P_{HWE}* value > 0.05). The genetic markers were subjected to allelic (*P_{CCA}*) and genotypic (*P_{CCG}*) case-control comparisons.

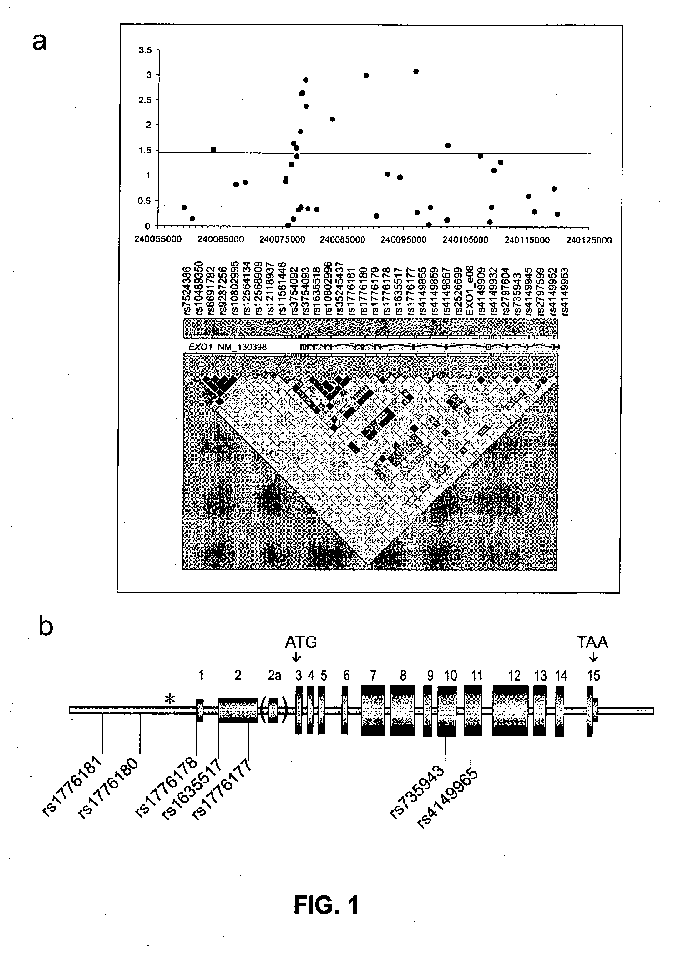

The cSNP with the highest significance, rs735943, was localized in exon 10 of the *EXO1* gene (*P_{CCA}* of 8.16 x 10⁻⁴ and *P_{CCG}* 0.0012; Table 1). An additional association signal (rs4149965) was present in exon 11 of the same gene (Table 1). Two other nominally significant cSNPs, which mapped to two different genes, were obtained from the analysis. Since their *P_{CCA}* or *P_{CCG}* (0.08 > *P* > 0.01) were much higher than the corresponding values for the *EXO1* cSNP rs735943 (Table 4), they were not further studied. As the *EXO1* lead SNP rs735943 was identified as the top-ranking marker and was additionally supported by another signal in a neighbouring exon, the *EXO1* gene was subjected to more in-depth investigations. Given the limited availability of centenarian samples worldwide, it was decided to subject only the functionally relevant SNPs to replication. In the following, therefore mutation detection, fine-mapping and functional studies were applied in order to identify and verify the polymorphisms underlying the association. Sequencing of all known exons, exon-intron boundaries and the promoter region in *EXO1* in 47 centenarians revealed several known and one novel polymorphism that were included in the subsequent fine-mapping, using 43 SNPs that span the entire gene (Table 5). Fourteen of these SNPs covered the extent of the association signal (Figure 1a), of which seven are located in potentially functional regions (Table 2a and Fig. 1b).

Referring to Fig. 1a an overview of the *EXO1* region on chromosome 1 is given. The physical position of all genotyped SNPs (y-axis) with their allele-based *P* values (-log10; x-axis) and a schematic representation of the gene structure for NM_130398 are shown. The significance threshold of *P* = 0.05 is indicated. Coordinates refer to NCBI genome assembly build 36. The LD plot of the locus (r²) was generated with Haploview 3.32 using the genotypes of German control individuals. For more information on the novel SNP EXO1_e08 see Table 5.

### Seven associated EXO1 SNPs have potentially functional relevance

When analyzing these potential functional SNPs separately by logistic regression with adjustment for *APOE,* the only gene consistently shown to influence life expectancy (Christensen et al. 2006), all seven SNPs showed an even stronger effect than without adjustment (Table 2a). No significant interactions between any two SNPs or between a SNP and *APOE* were observed. Since allelic variants may potentially influence the longevity phenotype in men and women differently (Franceschi et al. 2000; Candore et al. 2006), a sex-stratified analysis was additionally performed. In men, no significant associations were detected (data not shown), which is possibly due to a lack of power because of the small number of males investigated (79 centenarians vs. 77 controls). In women, all SNPs apart from one, showed stronger effects (Table 2b) than in the combined analysis (Table 2a).

With reference to Fig. 1b an illustration of the *EXO1* gene model with the positions of the potentially functionally relevant SNPs is given. *EXO1* has several isoforms, here the inventors refer to NM_130398 that contains 16 exons, 13 of which cover the coding region (indicated by big boxes), three exons (exon 1, exon 2 and alternative exon 2a) span the 5'UTR, and the last exon (nominal exon 15) contains coding sequence and the 3'UTR (small boxes). The seven SNPs analyzed in this study are located within functionally relevant regions: two SNPs in the 5'proximal promoter region (rs1776181A/G, rs1776180C/G), three SNPs in the 5'UTR (rs1776178A/G, rs1635517G/A, rs1776177C/T) and two non-synonymous SNPs in the coding region (rs735943A/G resulting in amino acid substitution H354R; rs4149965G/A resulting in V458M). ATG: start codon in nominal exon 3, TAA: stop codon in nominal exon 15. The position of the transcriptional start site as predicted by the Eponine prediction program is indicated by an asterisk (*).

To evaluate which of the seven associated SNPs has an impact on the expression or function of the EXO 1 protein, various *in vitro* assays were applied.

### Coding SNPs do not influence DNA repair capacity of EXO1 after UV B treatment

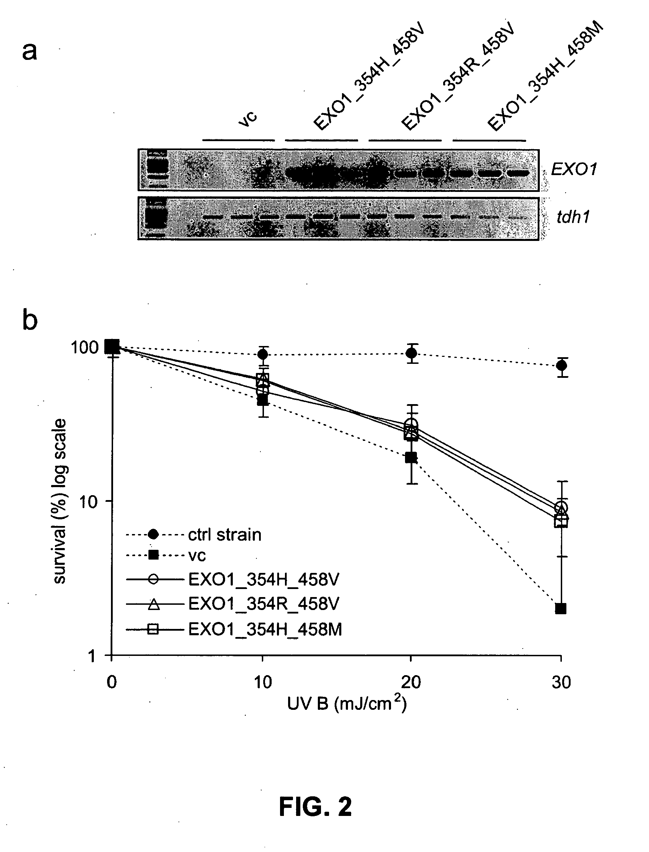

Human EXO1 protein can complement its yeast homologue in preventing DNA damage caused by UV B radiation (Qiu et al. 1999). Therefore, *S*. *cerevisiae* yeast strains deficient for yeast *exo1* and *rad51*, another exonuclease belonging to the same gene family, represent well-established models to analyze human EXO1 function. To assess the effect of the two *EXO1* cSNPs, the inventors over-expressed human EXO1 protein with either variant (H354R resulting from rs735943 A/G and V458M resulting from rs4149965 G/A) in UV B-sensitive FDER yeast cells (Δ*exo1*/Δ*rad51)*. All EXO1 proteins in this study were able to desensitize yeast cells from DNA damage-induced cell death (Fig. 2).

FIG. 2 shows the results of experiments, wherein yeast cells with genetic deficiency for *exo1* and *rad51* (Δ*exo1*/Δ*rad51*) were used to establish three independent clones for the human EXO1 protein variants EXO1_354H_458V, EXO1_354R_458V and EXO1_354H_458M. Fig. 2a shows that the insertion of the constructs into yeast genome was tested by PCR using primers for human *EXO1* or yeast glyceraldehyde-3-phosphat dehydrogenase (*tdh1*) as control. Fig. 2 shows that cells were spread on growth plates and exposed to different UV dosages to induce DNA damage. After 48 h, colonies were counted to calculate the survival rate depending on UV B dose. All EXO1 protein variants examined lead to a partial rescue of yeast cells from DNA damage-induced cell death (ctrl strain: UV B-insensitive S. *cerevisiae* MaV203, vc: Δ*exo1*/Δ*rad51* strain transformed with empty expression vector pDB20 as vector control (vc). Data represent mean ±SD of independent experiments (n=3) with three independent clones for each construct.)

No quantitative differences were observed between the three protein variants in their ability to protect yeast cells from UV B-induced DNA damage. The capacity of the human EXO1 protein variants to facilitate mutation avoidance is consistent with our previous findings using the same experimental setup (Qiu et al. 1999). It was concluded that the *EXO1* cSNP alleles do not influence the ability to repair UV-induced DNA damage. However, given the multiple roles of EXO1 in the maintenance of genomic stability, the possibility cannot be excluded that the two analyzed cSNPs may be functionally relevant in a different context than the one tested here.

### 5'UTR haplotypes of EXO1 do not show differential impact on reporter gene activity

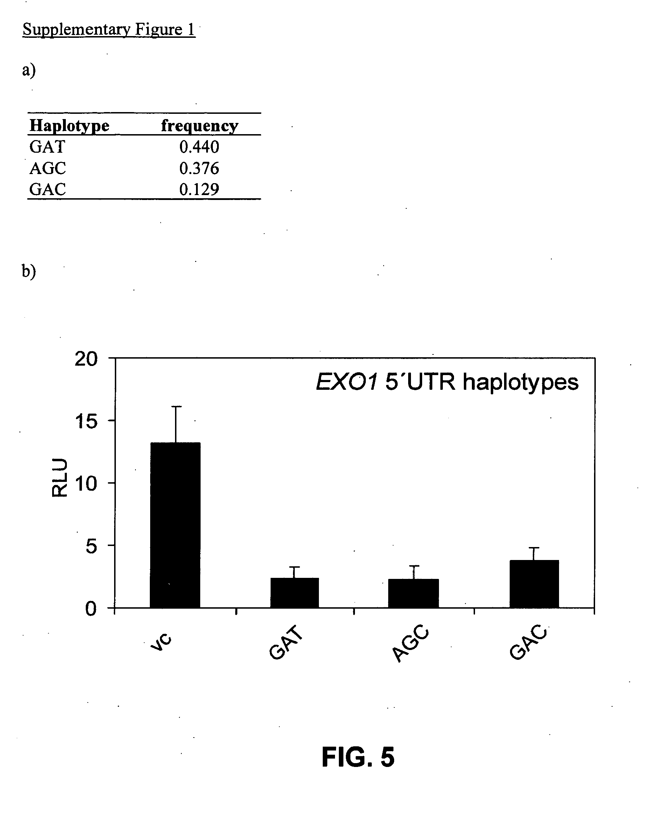

The inventors of the present application analyzed the effect of the four most common *EXO1* 5'UTR haplotypes observed in the present samples (representing the haplotypes AGC, GAT and GAC with regard to rs1776178, rs1635517 and rs1776177, see Fig. 5a) by inserting them in front of a heterologous reporter gene and performing dual luciferase assay with HeLa cells.

Fig. 5 illustrates the analysis of *EXO1* 5'UTR haplotypes. Fig. 5a shows that haplotypes are defined by the SNP order rs1776178, rs1635517 and rs1776177. The r2 values for the three SNP pairs range between 0.452 and 0.897. Only common haplotypes are presented with a frequency > 3% in control individuals. Fig. 5b shows the dual luciferase assay for analysis of 5'UTR haplotypes. Haplotype-specific constructs were established using reporter gene vector pGL3control as described in Supplementary Methods. All haplotypes tested resulted in a decrease of reporter gene activity compared to empty vector (vc). No significant differences were observed between the haplotypes. RLU: relative light units, vc: vector control.

Insertion of all four *EXO1* 5'UTR haplotypes reduced expression of the reporter gene by a factor of 4-6, thus arguing for an inhibitory role of this regulatory region in RNA processing and/or translation efficiency. However, the four haplotypes did not differ significantly from each other (Fig. 5b), indicating that the analyzed 5'UTR variants do not influence posttranscriptional processing or translation efficiency of *EXO1*.

### SNP rs1776180 influences EXO1 promoter activity in an allele-specific manner

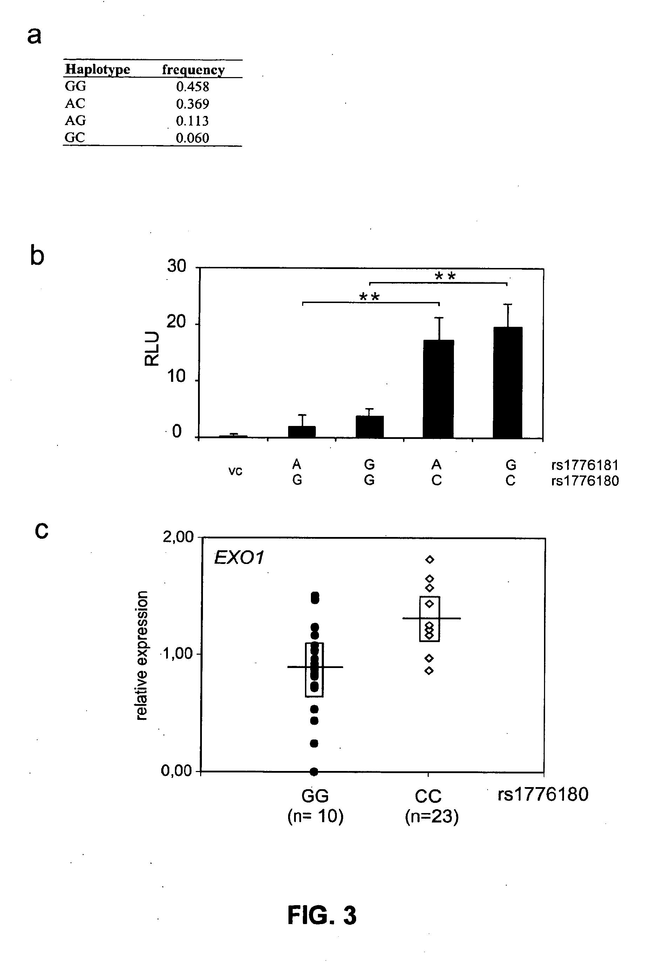

In order to investigate the functional impact of the promoter SNPs rs1776180 and rs1776181, reporter gene constructs based on the four possible haplotypes were established. Fig. 3a shows the frequencies of the four haplotypes observed in the control individuals (Haplotypes are defined by the SNP order rs 1776181 and rs 1776180; r² value for the SNP pair is 0.431).

Insertion of any promoter fragment increased reporter gene activity by a factor of up to 90 compared to empty vector. More importantly, our data indicate a remarkable influence of the rs 1776180 alleles on *EXO1* promoter activity. Fig. 3b shows results of dual luciferase assays with reporter gene constructs representing *EXO1* promoter haplotypes to quantify promoter activity. HeLa cells or HEK293 cells (not shown) were transfected as described and used for quantification of constitutive promoter activity. As vector control (vc) empty vector pGL3Enhancer was used. Both haplotypes containing the C allele of rs 1776180 showed a 6-9 fold higher activity than the corresponding haplotypes carrying the G allele (**; *P* < 0.01 for both constructs) irrespective of the allele at rs1776181. Data are expressed as relative light units (RLU) and represent mean values +SD (n=3).

Both haplotypes containing the C allele of rs 1776180 showed a 6-9 fold higher activity than the corresponding haplotypes carrying the G allele (*P* < 0.01 for both constructs) irrespective of the allele at rs 1776181. No significant impact on promoter activity was observed between the two constructs carrying either variant at rs 1776181. Thus, the C allele of rs 1776180 seems to be self-sufficient for enhanced promoter activity of *EXO1*. Interestingly, the C allele of rs 1776180 is statistically significantly over-represented in centenarians (Table 2a).

EXO1 *mRNA level is up-regulated in females carrying the C allele of SNP rs1776180* The above-described results prompted us to implement *ex vivo* analyses of *EXO1* expression in relation to the rs1776180 genotypes. No significant genotype-dependent *EXO1* mRNA regulation was observed in lymphoblast cell lines originating from male individuals. In contrast, *EXO1* transcript levels were significantly up-regulated (*P* = 0.034; 1.34-fold induction) in cell lines originating from females with the CC genotype (n =10) when compared to women with the GG genotype (n=23) (see RT-PCR data in Fig. 3c, which shows genotype-specific expression of *EXO1*. Real-time PCR was used to quantify the relative expression level of *EXO1* mRNA in human lymphoblast cells in relation to the rs1776180 genotypes. Data represent the expression level of *EXO1* normalized to housekeeping gene β-actin and are expressed as median ± SD. In females, the genotype CC (n=10) of rs1776180 is associated with a 1.39 fold higher relative *EXO1* expression compared to genotype GG (n=23)). In addition, an increased *EXO1* mRNA level was found in females with the CC genotype relative to female carriers of the G allele (n=59; *P* = 0.016; 1.37-fold induction, not shown).

These data support the notion of a female-specific functional effect, as already observed in the genetic analysis.

### Allele-specific binding of E47 contributes to transcriptional repression of EXO1

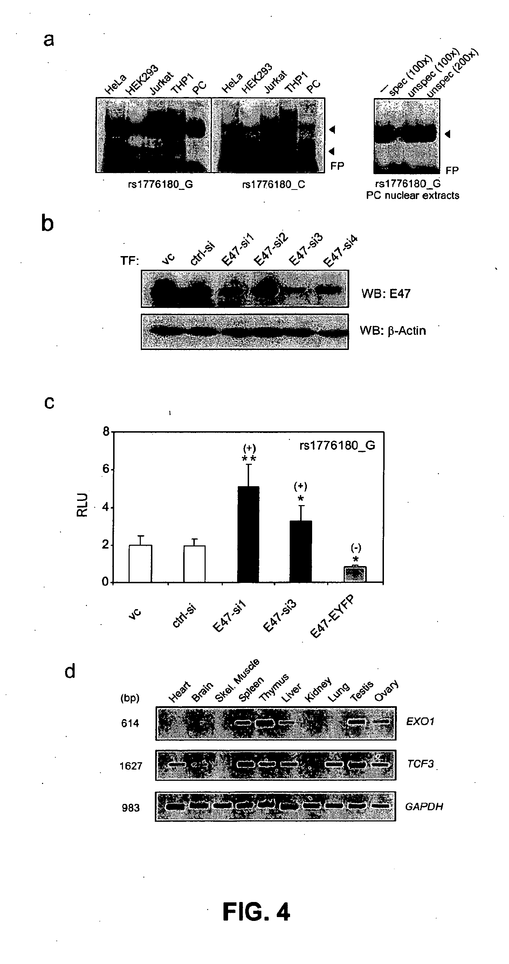

To shed more light on the functional mechanisms behind the allele-dependent promoter activity of *EXO1,* the inventors performed bandshift assays with oligonucleotides representing the region surrounding rs1776180 and a panel of nuclear extracts derived from different human cell lines. It was detected a notable increase in protein binding for the G allele of rs1776180 with all extracts tested (cf. Fig. 4a, left panel). Specificity of binding was demonstrated by competition experiments (cf. Fig. 4a, right panel).

In Fig. 4 showing that transcription factor E47 acts as transcriptional repressor of *EXO1*, Fig. 1a shows the presence of the G allele of rs 177618 results in increased binding of nuclear proteins. Bandshift assays were performed with oligonucleotides representing the G or C allele of rs 1776180, respectively, and nuclear extracts of HeLa, HEK293, THP1, Jurkat and pancreatic carcinoma cells. For all nuclear extracts analyzed, increased protein binding is observed for the G allele (left panel). Specificity of binding was shown for the G allele by competition experiments using unlabelled specific oligonucleotide or unspecific oligonucleotide at 100- or 200-fold molar excess (right panel). FP: free probe, PC: pancreas carcinoma, arrowheads: specific bandshifts.

These findings are indicative of a potential negative regulator of transcription as a determining factor for altered promoter activity. The Consite transcription factor binding site prediction tool showed that the C allele of rs 1776180 results in loss of a specific binding site for transcription factor E47. E47 is encoded by the gene *TCF3* (alias *E2A*). Analysis of *TCF3* with SNPs covering most of the allelic variation at the locus provided no evidence for association with longevity (Table 7). For further analysis of functional crosstalk between E47 and *EXO1* regulation, RNAi mediated gene knock-down of E47 and transient over-expression of E47-EYFP fusion protein were used.

Fig. 4b shows RNAi-mediated knock-down of E47 protein level. HeLa cells were transiently transfected with pSUPER constructs carrying independent target sequences directed against E47 (si1-si4) or unspecific control sequence (si-ctrl). Proteins were extracted 48 h later and subjected to Western blot analysis using anti-E47 antibody. Constructs si1 and si3 resulted in remarkable reduction in E47 protein expression and were chosen for use in dual luciferase assay. Fig. 4c shows dual luciferase assay with pGL3Enhancer construct representing promoter haplotype rs_176181A / rs1776180_G. Gene silencing of E47 by shRNA constructs si1 and si3 increased promoter activity significantly while unspecific control shRNA showed no effect in comparison to vector control (vc; empty pGL3Enhancer). In addition, over-expression of E47-EYFP fusion protein impaired reporter gene expression. (+) indicates significant up-regulation versus ctrl-si, (-) indicates significant down-regulation versus ctrl-si (*; P<0.05, **; P<0.01). Fig. 4d shows overlapping tissue expression patterns of *EXO1* and *E47*/ *TCF3.* Commercially available human cDNA tissue panels were used to analyze tissue distribution by RT-PCR (see below "Supplementary Methods" for details). Overlapping expression can be detected in spleen, thymus, liver, testis and ovary. Expression of *GAPDH* is shown as control.

As expected, knock-down of E47 by RNAi resulted in a statistically significant increase in *EXO1* promoter activity (Fig. 4b) while over-expression of E47 decreased luciferase activity (Fig. 4c). Since this novel regulatory mechanism is only relevant in tissues and cell types expressing both *EXO1* and *E47*/*TCF3,* the tissue distribution pattern of both genes was analyzed. As shown in Fig. 4d, *EXO1* is strongly expressed in thymus, spleen and testis and showed a weaker, but yet detectable, expression in brain, liver and ovary. Most interestingly, *E47*/*TCF3* mRNA showed a remarkably similar expression pattern with highest signals in spleen, thymus, liver, and testis and lower expression in heart, brain and ovary. Thus, concomitant expression of *EXO1* and *E47*/*TCF3* was most pronounced in lymphatic tissues and the reproductive tract.

Taken together, our data indicate a major impact of allelic variants of promoter SNP rs 1776180 on *EXO1* transcriptional regulation and suggest an important and hitherto undescribed role for E47 as repressor of *EXO1* expression.

### Association of promoter SNP rs1776180 with the longevity phenotype confirmed in female French centenarians

To obtain independent verification of the association between the functional polymorphism rs1776180 and longevity, the SNP in a collection of 450 French centenarians and 109 younger individuals whose age range was comparative to that of the German controls were analyzed. For the replication only females were considered since the effect of the marker was apparent only in German women (Table 2b and Fig. 3c). In the French, the analysis with adjustment for *APOE* confirmed the association signal *(P_{APOE}* = 0.044) (Table 2c). In both populations the allele and genotype frequencies were very similar and there was an enrichment of the C allele in the centenarians compared to the controls. Thus, the combined genetic and functional analyses point to a pivotal role of rs 1776180 in the allele-specific regulation of *EXO1* that in turn influences the ability of surviving to exceptional old age.

### DISCUSSION

The inventors of the present application have identified the exonuclease 1 gene (*EXO1*) as a novel susceptibility locus for exceptional life expectancy in humans. The associated promoter SNP rs1776180 was found to be functionally relevant by influencing *EXO1* expression. Interestingly, the association appeared stronger in females than in males. The promoter region surrounding rs1776180 contains a binding site for E47 only when the G allele is present. The C allele is enriched in centenarians and leads to a loss of the binding site. Since E47 acts as a repressor of *EXO1* transcription, the substitution of G by C at rs 176180 consequently results in a higher promoter activity. Supportive evidence was obtained from *ex vivo* analysis of *EXO1* inRNA levels in lymphoblast cell lines that showed a statistically significant increase in gene expression in female individuals.

EXO1 represents a 5'-3' exonuclease that participates in DNA mismatch repair (MMR) and other DNA metabolic pathways affecting genomic stability, including mitotic and meiotic recombination, double-strand break and UV damage repair as well as telomere integrity (Qiu et al. 1999; Liberti & Rasmussen 2004). EXO 1 physically interacts with the WRN protein that is encoded by the gene implicated in Werner Syndrome, one of the human premature aging diseases (Sharma et al. 2003). WRN is crucial for the maintenance of overall genomic stability and is, like EXO1, also required for DNA repair and telomere metabolism (Lombard et al. 2005). The binding of both proteins causes a strong activation of EXO1 (Sharma et al. 2003). It has been suggested that WRN stimulation of EXO1 may be important for the replication and stability of telomeres and/or the activation of DNA damage and signal checkpoint pathways in response to shortened telomeres (Sharma et al. 2003). That EXO1 is involved in these pathways has been shown in both telomere-dysfunctional yeast and mouse models (Maringele & Lydall 2002; Schaetzlein et al. 2007).

The important role of EXO1 in controlling life expectancy has previously been reported in two different knock-out mice strains that are homozygous for a deletion in the nuclease domain of the gene (*Exo1* ⁻/⁻). The animals were viable, but their lifespan was significantly shortened (Wei et al. 2003; Schaetzlein et al. 2007). The first *Exo1 ⁻*/*⁻* knock-out mice were generated on a mixed genetic background. The animals had defects in their MMR system and a slightly higher rate of spontaneous cancers (Wei et al. 2003). These results raise the possibility that the beneficial influence of an increased *EXO1* activity on lifespan could lie in mutation avoidance and, therefore, in the prevention of tumorigenesis. However, a recent study has shown that the Exo1 inactivation in knock-out mice of a genetic background different from the previous one (Wei et al. 2003) impeded considerably their survival without a significant rise in cancer formation, indicating that other factors contribute to the lifespan reduction in *Exo1⁻l⁻* mice (Schaetzlein et al. 2007). Similarly, also in humans it is not clear whether mutational changes in *EXO1* efficiency affect carcinogenesis (reviewed in Liberti & Rasmussen 2004). The possible links between *EXO1* variation, cancer and life expectancy need to be further elucidated.

The present data implicate E47 as a repressor of *EXO1* transcription. E47 (encoded by the gene *TCF3*/*E2A*) belongs to the family of basic helix-loop-helix transcription factors and is involved in the regulation of cell commitment, growth and differentiation (Slattery et al. 2007). A recent study using TCF3/E2A-deficient murine lymphoma cell line and hierarchical cluster analysis of DNA microarray data identified E47-dependent gene expression patterns contributing to cell cycle progression, lipid metabolisms, stress response and cell survival (Schwartz R et al., 2006). Interestingly, there are several lines of evidence linking E47 to aging processes: E47 mRNA level and activity are diminished in spleen and bone marrow of aged mice compared to young mice (Frasca D. et al., 2003). In addition, impaired E47 activity has been shown to correlate with reduced immunoglobulin class switch in senescing B cells (Frasca D. et al., 2004). The contribution of E47 to cellular aging processes is further emphasized by the finding that gene silencing of E47 delays the onset of cellular senescence in human fibroblasts (Zheng W et al., 2004). Furthermore, E47 is critical for the development of lymphoid lineages. It has been detected in both mature B and T cells and its precursors (Murre 2005) and shows a high expression in tissues that are areas of active hematopoiesis, i.e. adult bone marrow (Liberti & Rasmussen 2004), spleen and thymus (Fig. 4d). The regulatory mechanism of E47 on EXO1 expression observed in this study implies that the concomitant expression of both genes is required for the C allele of rs 1776180 to be advantageous. Strikingly, our data reveal an overlap of their expression profiles in a number of tissues, including liver, thymus, spleen, testes and ovary. Concomitant expression of both genes in reproductive organs favours the view that allele-specific expression of EXO1 might participate in the protection of the germ line from DNA damage during meiotic recombination. It is still unclear whether mutation avoidance during germ cell maturation is linked to higher life expectancy. On the other hand, overlapping expression of E47/TCF3 and EXO1 was also detectable in lymphatic tissues, arguing for their specific role in hematopoiesis and regulation of the immune response. This scenario is supported by the observation that both E47 and EXO1 participate in, for instance, class-switch recombination and the generation of antibody diversity during B-cell development (Liberti & Rasmussen 2004; Murre 2005). Thus, it is hypothesized that an enhanced EXO1 activity could mitigate defects in humoral and cell-mediated immunity that arise during aging.

In the present study, there was observed a probable female-specific association of the *EXO1* promoter SNP with longevity. This finding is not surprising as gender accounts for important differences in the frequency of various age-related diseases and is also thought to play a role in the genetic influence on the ability to reach an exceptional old age. There is a distinct prevalence of women among long-lived individuals, reaching up to 85% in some populations of centenarians and super-centenarians (Perls & Terry 2003). It has been suggested that the mortality rates in men and women follow different trajectories during aging and that a complex interaction of environmental and genetic factors is likely to affect the gender-specific probability of achieving a long life (Franceschi et al. 2000; Candore et al. 2006).

The present results for the *EXO1* gene are another example for the importance of centenarians in genetic longevity research. The oldest old individuals belong to the top percentiles of their respective birth cohort-specific age distributions and have outlived most of their peers by several decades. Since the genetic contribution to life expectancy is strongest at very advanced ages (Hjelmborg et al. 2006), centenarians may be particularly enriched for beneficial variants in longevity enabling genes (Perls et al. 2002). This hypothesis is strengthened by the findings in the present study, which demonstrate that variation in *EXO1* leads to a higher gene expression and a survival advantage at very old age. Hence, *EXO1* could be considered a novel longevity enabling gene.

### EXAMPLE

### Methods

### Study participants

The German DNA collections and the recruitment procedures where reported in detail elsewhere (Nebel et al. 2005). The sample comprised 381 centenarians (100 - 110 years at ascertainment; mean: 101.3 years); 79% of them were female. The centenarian case sample was specifically matched to a group of 409 younger controls (60 to 75 years; mean: 66.6 years) by considering gender and geographic origin within the country. Results were not corrected for potential population substructure as centenarians and controls were matched by region and gender and as very low F_{ST} values have been reported between different populations in Germany (Steffens et al. 2006).Moreover, the validity and efficacy of our longevity study populations have previously been demonstrated (Nebel et al. 2005). The French sample set consisted of 450 female centenarians (mean age: 103.7 years) and 109 female controls (aged 60 to 70 years; mean age: 64.5 years) as described previously (Blanché et al. 2001). DNA was isolated from blood samples of all participants using standard methods. All subjects gave informed, written consent prior to participation. The study was approved by the respective institutional review committees of the participating centers.

### Gene and cSNP selection

The investigated 92 coding SNPs (Table 3) are located in 49 genes involved in DNA repair processes and represent a subset of the cSNP panel described elsewhere (Hampe et al. 2007; see URL below).

### Genotyping

Genotyping was performed using the SNPlex^{™} Genotyping System and TaqMan^{®} SNP Genotyping Assays (Applied Biosystems, Foster City, USA).

### Statistical analysis

Single-marker case-control analyses were performed with Genomizer (see URL below). Tagging SNPs were selected based on the HapMap genotypes of Europeans (see URL below) and the pairwise tagging option implemented in the Haploview v3.32 program (see URL below) (minor allele frequency 0.03, pairwise r² >= 0.8, *P*_{HWE} > 0.01; for tag SNPs, see Table 5). Haploview was also used for determining the linkage disequilibrium (LD) measure r² between SNPs in controls. Both the seven *EXO1* SNPs (Table 2a) typed in the German sample and the functionally relevant SNP rs1776180 typed in the French sample set were analyzed by logistic regression using a multiplicative effects model for the genotypes with the statistics program R (see URL below), adjusting for *APOE. P* values smaller than 0.05 were considered statistically significant.

### Mutation detection

Sequencing of the exons, intron-exon boundaries and the promoter region of *EXO1* was done in 47 centenarians with the BigDye^{®} Chemistry (Applied Biosystems) (for primer sequences, see Table 7). The *EXO1* promoter was localized by means of the 'First Exon and Promoter Prediction Program for Human DNA' (see URL below). Sequencing traces were inspected for the presence of SNPs and indels using novoSNP (Weckx et al. 2005).

### Cell culture and transfection of human cell lines

Human cell lines were cultured under standard conditions as described in Supplementary Methods (see below). Transfections were performed using Fugene 6™ (Roche, Basel, Switzerland) according to the manufacturer's manual.

### Construction of plasmids

Construction of plasmids for the over-expression of human EXO1 protein variants (due to the different alleles at the two cSNPs rs735943 A/G [H354R] and rs4149965 G/A [V458M]) in yeast and quantitative analysis of the *EXO1* 5'UTR and 5'promoter region was performed using standard cloning procedures as described in the Supplementary Methods (see below).

### Yeast strains and DNA damage treatment

The UV B-sensitive yeast strain FDER (Δ*exo1*/Δr*ad51*; Qiu et al. 1999) was used for generation of over-expression clones for the human EXO1 protein variants using standard yeast transformation techniques. For quantification of UV B-induced DNA damage, yeast cells were spread on growth plates, exposed to different UV dosages ranging from 0 to 40 mJ/cm2 and incubated for 48 h at 30°C. Further details are provided in the Supplementary Methods (see below).

### Dual luciferase assay

For quantitative analysis of the 5'UTR and 5'proximal promoter region, pGL3 constructs were used for dual luciferase reporter gene assay as described before (Till et al. 2005). Further information is provided in the Supplementary Methods (see below).

### Electrophoretic mobility shift assay

Preparation of nuclear extracts from human cell lines and bandshift assays were performed as previously described (Arlt et al. 2004). Sequences of oligonucleotides used and further details are outlined in the Supplementary Methods (see below).

### Analysis of transcription factor binding sites

For analysis of *EXO1* promoter regions containing the allelic variants at the SNPs rs1776181 and rs1776180, the sequences were analyzed for potential transcription factor binding sites using Consite (see URL below).

### Quantification of EXO1 transcript levels in lymphoblast cell lines

A total of 123 lymphoblast cell lines were obtained from heparinized blood samples of unrelated healthy donors (from the Popgen biobank; Krawczak et al. 2006) using standard EBV transformation methods. DNA was isolated by standard methods and was genotyped for rs1776180 using a TaqMan^{®} SNP Genotyping Assay (Applied Biosystems, Foster City, USA). Total RNA was extracted and processed with the Qiagen RNeasy Kit according to the manufacturer's guidelines (Qiagen, Hilden, Germany). Real-time PCR was performed using a 7900HT Fast Real Time PCR-System (Applied Biosystems, Foster City, USA). Expression levels of *EXO1* (assay Hs00243513_ml) were calculated with the standard curve method (Livak & Schmittgen 2001). Significance was determined using the Mann-Whitney U-test.

### Analysis of tissue-specific expression patterns

To analyze the tissue expression pattern of EXO1 and E47 / TCF3, commercially available human cDNA tissue panels (Clontech) and standard PCR techniques were used. For primer sequences and further information, see below "Supplementary Methods".

Supplementary Methods

### Cell Culture

Human embryonal kidney HEK293 (ACC 305), cervical carcinoma HeLa cells (ACC 57), human acute monocytic cell line THP-1 (ACC16) and human T cell leukemia Jurkat cells (AC282) were purchased from the German Collection of Microorganisms and Cell Cultures (DSMZ, Braunschweig, Germany) and cultured in DMEM (HEK293) or RPMI1640 (THP-1, Jurkat, HeLa, all media from PAA Laboratories, Paschberg, Austria). Pancreas carcinoma cells (PC) were kindly provided by Ming-Sound Tsao (Ontario, Canada) and cultivated in RPMI. Media were supplemented with 10% fetal calf serum (FCS) and penicillin/streptomycin (each at 50 µg/ml), cells were grown in 5% CO2 at 37°C. Transfections were performed using Fugene 6™ (Roche, Basel, Switzerland) according to the manufacturer's manual.

### Construction of plasmids

Plasmids for overexpression of human EXO1 protein in yeast cells were generated by inserting full length *EXO1* cDNA between two *Not* I sites of expression vector pDB20. Constructs for analysis of *EXO1* 5'UTR were designed by amplifying 584 bp of the 5'UTR region from a human cDNA tissue panel (Clontech, Palo Alto, CA) and insertion into *Hind* III and *Nco* I sites of pGL3control (Promega, Madison, WI). Constructs for analysis of *EXO1* 5'proximal promoter were designed by amplifying 1199 bp of the promoter region from human genomic DNA and insertion into *Nhe* I and *Xho* I sites of pGL3Enhancer (Promega). Allel-specific constructs were established by site-directed mutagenesis using the QuickChange mutagenesis kit (Stratagene, La Jolla, CA). The overexpession construct pMyc-E47-EYFP was kindly provided by Naihe Jing (Shanghai Institutes for Biological Sciences, Shanghai, China). For RNAi-mediated knock-down of *E47*/*TCF3* expression, four independent target sequences (E47_sil (SEQ ID NO: 39): TGAACCAGCCGCAGAGGAT; E47_si2 (SEQ ID NO: 40): GAAGGTCCGGAAGGTCCCG; E47_si3 (SEQ ID NO: 41): GGTGTCAGGTGTGGTTGGA; E47_si4 (SEQ ID NO: 42): GCCCA-CAACCCCGCCGGGC) directed against *E47*/*TCF3* mRNA were inserted into pSUPER.Neo+GFP (OligoEngine, Seattle, WA) and checked for silencing of E47 protein expression by Western blotting. Two constructs with the highest efficiency to knock down E47 expression were chosen for further experiments.

### Yeast strains and DNA damage treatment

The UV B-sensitive yeast strain FDER (*Δexo1*/Δr*ad51*) was used for generation of overexpression clones for the allele specific variants of *EXO1* applying standard yeast transformation techniques. Insertion of the constructs into yeast genome was tested by PCR with primers for human *EXO1* or yeast glyceraldehyde-3-phosphat dehydrogenase (*tdh1*) as control. The following primers were used: Sc_TDH1-225F (SEQ ID NO: 43) CCAAGAAAGA-GACCCAGCTAAC, Sc_TDH1_855R (SEQ ID NO: 44): CAAGAAATCAGAGGA-GACAACG, EXO1-996F (SEQ ID NO: 45): GATGCCCCACAATTGAAGG, EXO1-1510R (SEQ ID NO: 46) CGTGCTACATCTGTGTCAACC. Three independent clones were generated for each construct, clones containing empty expression vector (pDB20) served as negative control, UV B-insensitive yeast strain MaV203 served as specificity control. For quantification of UV B-induced DNA damage, yeast cells were spread on growth plates, exposed to different UV dosages ranging from 0 to 40 mJ/cm2 (UVB light source; Phillips TL 20/W12, Eindhoven, Netherlands) and incubated for 48 h at 30°C. The number of resulting colonies was counted and calculated as percentage of untreated controls.

### Dual luciferase assay

For quantitative analysis of the 5'UTR and 5'proximal promoter region, pGL3 constructs were used for dual luciferase reporter gene assay as described before (Till A. et al. 2005). In short, HeLa cells or HEK293 (not shown) were transfected with pGL3 reporter gene constructs in combination with pRL-TK reference plasmid (Promega). For some experiments, cells were cotransfected with pSUPER shRNA constructs targeting E47 or overexpression construct pMyc_E47_EYFP. 24 h after transfection, cells were lysed in Passive Lysis Buffer (Promega) and assayed for reporter gene activity using a MicroLumatPlus Luminometer (Berthold, Germany).

### Electrophoretic mobility shift assay

Preparation of nuclear extracts from human cell lines and bandshift assays were performed as previously described (Arlt A. et al. 2004). The sequence of the oligonucleotides representing the allelic variants of the *EXO1* promoter were EXO1P_rs1776180_C (SEQ ID NO: 1): 5'-CGGGCCGGCAGCAGCGGCTGCAGTCGTATGGCAGTGAGCCGCTGTCTACC -3' and EXO1P_rs1776180_G (SEQ ID NO: 2): 5'- CGGGCCGGCAG-
CAGCGGCTGGAGTCGTATGGCAGTGAGCCGCTGTCTACC-3' (The affected nucleotide position is underlined). Specific competition of binding was tested by addition of unlabelled oligonucleotide EXO1P_rs1776180_G at 100x molar excess, unspecific competition was tested by addition of unlabelled oct-1 oligo (Promega) at 100x and 200x molar excess as described.

### Western blotting

HeLa cells transiently transfected with pSUPER constructs targeting *E47*/*TCF3* were lysed in Laemmli buffer in the presence of protease inhibitors. Cleared protein extracts were subjected to SDS-PAGE and Western blotting was performed as previously described (Till, A. et al. 2005). E47 protein levels were detected using specific mouse anti-E47 antibody (G98-271, BD Biosciences, San Jose, CA), equal loading was demonstrated by visualization of β-Actin (mouse-anti-Actin antibody purchased from Sigma-Aldrich Corp., St. Louis, MO).

### Analysis of tissue-specific expression pattern

To analyze the tissue expression pattern of *EXO1* and *E47*/*TCF3* commercially available human cDNA tissue panels (Clontech) and standard PCR techniques were used. The following primers were used: For analysis of human EXO1: EXO1-996F (SEQ ID NO: 45) GATGCCCCACAATTGAAGG, EXO1-1510R (SEQ ID NO: 46) CGTGCTACATCTGTGTCAACC; for E47/TCF3: TCF3-266F (SEQ ID NO: 47) CCTCTCTTCATC-CACATTCCTG, TCF3-1893R (SEQ ID NO: 48) CCACACCTGACACCTTTTCCTC. Housekeeping gene *GAPDH* was used as control and amplified using the following primers: GAPDH_F (SEQ ID NO: 49) TGAAGGTCGGAGTCAACGGATTTGGT, GAPDH_R (SEQ ID NO: 50) CATGTGGGCCATGAGGTCCACCAC.

### Industrial Applicability

The present invention is particularly useful for the screening of individuals having a nucleotide sequence associated with exceptional life expectancy. Furthermore, it is useful for preparing a pharmaceutical composition for germ line protection and the treatment of infectious and malignant diseases and diseases related to aging. Moreover, it will be appreciated that the present invention is useful for screening of substances affecting the expression of the EXO1 gene by using transcription factor E47 as a competitive binding agent. Finally, transcription factor E47 itself might serve as a basis for therapy, e.g. genetic targeting via RNA interference.

**Table 1: Association statistics for two coding SNPs in EXO1 (German sample)**

| **dbSNP ID** | **MAF controls** | **corresponding AF centenarians** | ***P*_{CCA}** | ***P*_{CCG}** |
|---|---|---|---|---|
| rs735943 | 0.415 | 0.499 | 8.16 x 10⁻⁰⁴ | 0.0012 |
| rs4149965 | 0.279 | 0.229 | 0.0250 | 0.0504 |

| | | | | |
|---|---|---|---|---|
| MAF: minor allele frequency. AF: allele frequency. *P*_{CCA}: *P* value obtained from an allele-based case-control χ² test. *P*_{CCG}: *P* value obtained from a genotype-based case-control χ² test. | | | | |

**Table 2a: Associated EXO1 SNPs of potentially functional relevance (German sample)**

| **No.** | **dbSNP ID** | **location** | **alleles** | **MAF controls (MA)** | **corresp. AF centenarians** | ***P*_{CCA}** | ***P*_{CCG}** | ***P_{APOE}*** | ***OR_{APOE}*** | **95% CI** |
|---|---|---|---|---|---|---|---|---|---|---|
| 1 | rs1776181 | promoter | A/G | 0.483 (A) | 0.545 | 0.0133 | 0.0449 | 0.0095 | 1.31 | 1.07-1.62 |
| 2 | rs1776180 | promoter | C/G | 0.430 (C) | 0.507 | 0.0024 | 0.0068 | 0.0016 | 1.39 | 1.14-1.72 |
| 3 | rs1776178 | 5'UTR | A/G | 0.412 (A) | 0.488 | 0.0022 | 0.0050 | 0.0019 | 1.39 | 1.12-1.72 |
| 4 | rs1635517 | 5'UTR | G/A | 0.425 (G) | 0.507 | 0.0012 | 0.0033 | 9.82 x 10⁻⁰⁴ | 1.41 | 1.15-1.75 |
| 5 | rs1776177 | 5'UTR | C/T | 0.472 (T) | 0.400 | 0.0041 | 0.0133 | 0.0021 | 0.72 | 0.59-0.89 |
| 6 | rs735943 | exon 10 | A/G | 0.415 (A) | 0.499 | 8.16 x 10⁻⁰⁴ | 0.0012 | 4.36 x 10⁻⁰⁴ | 1.45 | 1.18-1.79 |
| 7 | rs4149965 | exon 11 | G/A | 0.279 (A) | 0.229 | 0.0250 | 0.0504 | 0.0118 | 0.74 | 0.58-0.93 |

**Table 2b: Female subgroup analysis for EXO1 SNPs (German sample)**

| **No.** | **dbSNP ID** | **location** | **alleles** | **MAF controls (MA)** | **corresp. AF centenarians** | ***P*_{CCA}** | ***P*_{CCG}** | ***P_{APOE}*** | ***OR_{APOE}*** | **95% CI** |
|---|---|---|---|---|---|---|---|---|---|---|
| 1 | rs1776181 | promoter | A/G | 0.474 (A) | 0.558 | 0.0032 | 0.0131 | 0.0014 | 1.45 | 1.16-1.83 |
| 2 | rs1776180 | promoter | C/G | 0.427 (C) | 0.513 | 0.0023 | 0.0060 | 0.0012 | 1.47 | 1.16-1.85 |
| 3 | rs1776178 | 5' UTR | A/G | 0.409 (A) | 0.495 | 0.0022 | 0.0047 | 0.0013 | 1.47 | 1.16-1.85 |
| 4 | rs1635517 | 5' UTR | G/A | 0.423 (G) | 0.515 | 0.0011 | 0.0025 | 5.31 x 10⁻⁰⁴ | 1.52 | 1.19-1.92 |
| 5 | rs1776177 | 5' UTR | C/T | 0.476 (T) | 0.391 | 0.0026 | 0.0070 | 6.92 x 10⁻⁰⁴ | 0.67 | 0.53-0.84 |
| 6 | rs735943 | exon 10 | A/G | 0.405 (A) | 0.502 | 5.90 x 10⁻⁰⁴ | 4.75 x 10⁻⁰⁴ | 1.66 x 10⁻⁰⁴ | 1.56 | 1.23-2.00 |
| 7 | rs4149965 | exon 11 | G/A | 0.273 (A) | 0.226 | 0.0509 | 0.1495 | 0.0426 | 0.84 | 0.71-0.99 |

**Table 2c: Replication result for the EXO1 promoter SNP rs1776180 in a French sample collection (females)**

| **No.** | **dbSNP ID** | **location** | **alleles** | **MAF controls (MA)** | **corresp. AF centenarians** | ***P*_{CCA}** | ***P*_{CCG}** | ***P_{APOE}*** | ***OR_{APOE}*** | **95% CI** |
|---|---|---|---|---|---|---|---|---|---|---|
| 2 | rs1776180 | promoter | C/G | 0.408 (C) | 0.481 | 0.0530 | 0.1568 | 0.0441 | 1.39 | 1.01-1.92 |

| | | | | | | | | | | |
|---|---|---|---|---|---|---|---|---|---|---|
| Legend to Table 2a-c MA: minor allele. MAF: minor allele frequency. *P*_{CCA}*: P* value obtained from an allele-based case-control χ² test. *P*_{CCG}: *P* value obtained from a genotype-based case-control χ² test. *P_{APOE}*: *P* value obtained by logistic regression analysis after adjustment for *APOE* under the multiplicative model. *OR_{APOE}:* odds ratio for attaining old age with the minor allele in controls as reference allele, adjusted for *APOE*; CI, 95% confidence interval for *OR_{APOE}.* | | | | | | | | | | |

**Table 3: List of the 92 cSNPs in 49 repair genes analyzed**

| **Locus** | **dbSNP ID** | **chromosome** | **position build 36** | **alleles** |
|---|---|---|---|---|
| | rs6494 | 14 | 77210108 | [A/T] |
| *APEX1* | rs1130409 | 14 | 19994994 | [G/T] |
| *ATM* | rs1800056 | 11 | 107643213 | [C/T] |
| *ATM* | rs1800057 | 11 | 107648666 | [G/C] |
| *ATM* | rs1800058 | 11 | 107665560 | [C/T] |
| *ATR* | rs28897764 | 3 | 143763988 | [A/G] |
| *ATR* | rs2227928 | 3 | 143764302 | [C/T] |
| *BRCA1* | rs1799967 | 17 | 38476501 | [A/G] |
| *BRCA1* | rs16942 | 17 | 38497526 | [A/G] |
| *BRCA1* | rs16941 | 17 | 38497961 | [A/G] |
| *BRCA1* | rs799917 | 17 | 38498462 | [C/T] |
| *BRCA1* | rs4986850 | 17 | 38498997 | [A/G] |
| *BRCA1* | rs1799950 | 17 | 38500007 | [A/G] |
| *BRCA2* | rs766173 | 13 | 31804480 | [G/T] |
| *BRCA2* | rs 144848 | 13 | 31804729 | [G/T] |
| *BRIP1* | rs4986764 | 17 | 57118129 | [C/T] |
| *CAMK1* | rs1052133 | 3 | 9773773 | [G/C] |
| *CSNK1A1L* | rs9576175 | 13 | 36577268 | [G/T] |
| *DCLRE1A* | rs3750898 | 10 | 115599905 | [G/C] |
| *DCLRE1B* | rs12022378 | 1 | 114249912 | [C/T] |
| *ENO3* | rs238239 | 17 | 4797326 | [C/T] |
| *ERCC4* | rs1800124 | 16 | 13949578 | [A/G] |
| *ERCC5* | rs1047769 | 13 | 102311945 | [A/G] |
| *ERCC5* | rs2227869 | 13 | 102313086 | [G/C] |
| *ERCC5* | rs17655 | 13 | 102326003 | [G/C] |
| *ERCC6* | rs2228529 | 10 | 50337111 | [A/G] |
| *ERCC6* | rs4253211 | 10 | 50348323 | [G/C] |
| *ERCC6* | rs2228527 | 10 | 50348375 | [A/G] |
| *ERCC6* | rs2228526 | 10 | 50348723 | [A/G] |
| *ERCC6* | rs2228528 | 10 | 50402286 | [A/G] |
| *EXO1* | rs735943 | 1 | 240096774 | [C/T] |
| *EXO1* | rs4149963 | 1 | 240102005 | [C/T] |
| *EXO1* | rs4149965 | 1 | 240102061 | [A/G] |
| *EXO1* | rs1047840 | 1 | 240108924 | [A/G] |
| *EXO1* | rs1776148 | 1 | 240109168 | [A/G] |
| *LGALS8* | rs1041935 | 1 | 234767480 | [C/T] |
| *LIG4* | rs1805388 | 13 | 107661592 | [C/T] |
| *MAN2B1* | rs1054487 | 19 | 12633165 | [C/T] |
| *MAN2B1* | rs1054486 | 19 | 12635208 | [G/C] |
| *MGMT* | rs12917 | 10 | 131396273 | [C/T] |
| *MLH1* | rs1799977 | 3 | 37028572 | [A/G] |
| *MLH3* | rs17782839 | 14 | 74583216 | [A/G] |
| *MSH3* | rs184967 | 5 | 80185737 | [A/G] |
| *MSH3* | rs26279 | 5 | 80204693 | [A/G] |
| *MSH4* | rs5745549 | 1 | 76151090 | [A/G] |
| *MSH5* | rs1802127 | 6 | 31837904 | [C/T] |
| *MUTYH* | rs3219484 | 1 | 45572743 | [A/G] |
| *NEIL2* | rs8191613 | 8 | 11674685 | [A/G] |
| *NEIL3* | rs7689099 | 4 | 178493907 | [G/C] |
| *NEIL3* | rs17064658 | 4 | 178494358 | [G/C] |
| *NEIL3* | rs13112358 | 4 | 178511744 | [C/T] |
| *NEIL3* | rs13112390 | 4 | 178511829 | [A/C] |
| *PMS1* | rs2066459 | 2 | 190416957 | [A/G] |
| *POLE* | rs5744751 | 12 | 131764068 | [C/T] |
| *POLH* | rs9333555 | 6 | 43689913 | [A/G] |
| *POLH* | rs6941583 | 6 | 43690069 | [A/T] |
| *POLI* | rs8305 | 18 | 50074803 | [A/G] |
| *POLQ* | rs2306211 | 3 | 122634474 | [A/G] |
| *POLQ* | rs3218634 | 3 | 122634502 | [G/C] |
| *POLQ* | rs1381057 | 3 | 122637664 | [C/T] |
| *POLQ* | rs532411 | 3 | 122669112 | [C/T] |
| *POLQ* | rs3218642 | 3 | 122690327 | [A/C] |
| *POLQ* | rs3218651 | 3 | 122690866 | [A/G] |
| *POLQ* | rs3218649 | 3 | 122691523 | [G/C] |
| *POLQ* | rs487848 | 3 | 122711650 | [A/G] |
| *RAD1* | rs1805327 | 5 | 34944634 | [A/G] |
| *RAD1* | rs1805328 | 5 | 34947671 | [G/C] |
| *RAD18* | rs373572 | 3 | 8930389 | [A/G] |
| *REV1* | rs3087399 | 2 | 99421590 | [A/G] |
| *REV1* | rs3087386 | 2 | 99421938 | [C/T] |
| *REV1* | rs3087403 | 2 | 99425302 | [A/G] |
| *REY3L* | rs3204953 | 6 | 111735319 | [A/G] |
| *REV3L* | rs462779 | 6 | 111802580 | [C/T] |
| *REV3L* | rs458017 | 6 | 111802784 | [C/T] |
| *RTEL1* | rs3848668 | 20 | 61763716 | [A/G] |
| *SIRT3* | rs28365927 | 11 | 226091 | [A/G] |
| *SNPH* | rs450739 | 20 | 1158647 | [C/T] |
| *SNPH* | rs203534 | 20 | 1162814 | [G/T] |
| *SRBD1* | rs3755072 | 2 | 45493838 | [C/T] |
| *SRBD1* | rs3755073 | 2 | 45493878 | [A/C] |
| *TBC1D1* | rs6811863 | 4 | 37638581 | [G/C] |
| *TP53BP1* | rs2602141 | 15 | 41511938 | [G/T] |
| *TP53BP1* | rs689647 | 15 | 41549488 | [C/T] |
| *TP53BP1* | rs560191 | 15 | 41555066 | [G/C] |
| *UTP6* | rs3760454 | 17 | 27246115 | [A/G] |
| *WDR33* | rs17534123 | 2 | 128238677 | [C/T] |
| *WRN* | rs2230009 | 8 | 31041477 | [A/G] |
| *WRN* | rs1800391 | 8 | 31058246 | [A/G] |
| *WRN* | rs2725362 | 8 | 31118822 | [G/T] |
| *XPC* | rs2227999 | 3 | 14174910 | [A/G] |
| *XRCC1* | rs25487 | 19 | 48747566 | [A/G] |
| *XRCC4* | rs28360135 | 5 | 82527430 | [C/T] |

**Table 4: Association statistics for nominally significant SNPs in other DNA repair genes (German sample)**

| **dbSNP ID** | **locus** | **chromosome** | **position build 36** | **MAF controls** | **corresp. AF centenarians** | ***P*_{CCA}** | ***P*_{CCG}** |
|---|---|---|---|---|---|---|---|
| rs130409 | *APEX1* | 14 | 19994994 | 0.45 | 0.51 | 0.01 | 0.01 |
| rs28360135 | *XRCC4* | 5 | 82527430 | 0.03 | 0.05 | 0.03 | 0.08 |

| | | | | | | | |
|---|---|---|---|---|---|---|---|
| MAF: minor allele frequency. AF: allele frequency. *P*_{CCA}: *P* value obtained from an allele-based case-control χ² test. *P*_{CCG}*: P* value obtained from a genotype-based case-control χ² test. | | | | | | | |

**Table 5: Fine mapping of the EXO1 region**

| **dbSNP ID** | **position build 36** | **major AF** | ***P*_{CCA}** | ***P*_{CCG}** |
|---|---|---|---|---|
| rs7524386 | 240059161 | 0.80 | 0.43 | 0.22 |
| rs10489350 | 240060454 | 0.82 | 0.72 | 0.36 |
| rs6691782 | 240063727 | 0.61 | 0.03 | 0.04 |
| rs9287256 | 240067411 | 0.59 | 0.15 | 0.08 |
| rs10802995 | 240068905 | 0.59 | 0.13 | 0.09 |
| rs12564134 | 240075450 | 0.59 | 0.13 | 0.09 |
| rs12568909 | 240075497 | 0.59 | 0.12 | 0.08 |
| rs12118937 | 240075930 | 0.78 | 0.95 | 0.94 |
| rs11581448 | 240076439 | 0.51 | 0.06 | 0.04 |
| rs3754092 | 240076695 | 0.95 | 0.72 | 0.72 |
| rs3754093 | 240076739 | 0.82 | 0.02 | 0.03 |
| rs1635518 | 240077197 | 0.52 | 0.03 | 0.05 |
| rs10802996 | 240077250 | 0.76 | 0.04 | 0.10 |
| rs35245437 | 240077630 | 0.92 | 0.47 | 0.53 |
| rs1776181 | 240077867 | 0.52 | 0.01 | 0.04 |
| rs1776180 | 240077967 | 0.57 | 0.002 | 0.007 |
| rs1776179 | 240078029 | 0.68 | 0.42 | 0.68 |
| rs1776178 | 240078157 | 0.59 | 0.002 | 0.005 |
| rs1635517 | 240078656 | 0.57 | 0.001 | 0.003 |
| rs1776177 | 240078772 | 0.53 | 0.004 | 0.01 |
| rs4149855 | 240079229 | 0.91 | 0.45 | 0.70 |
| rs4149859 | 240080696 | 0.98 | 0.46 | 0.46 |
| rs4149867 | 240083213 | 0.79 | 0.008 | 0.02 |
| rs2526699 | 240088753 | 0.59 | 0.001 | 0.001 |
| EXO1_e08* | 240090494 | 0.99 | 0.61 | ** |
| rs4149909 | 240090521 | 0.97 | 0.64 | 0.61 |
| rs4149932 | 240092389 | 0.99 | 0.09 | ** |
| rs2797604 | 240094409 | 0.53 | 0.11 | 0.22 |
| rs735943 | 240096774 | 0.59 | 0.0008 | 0.001 |
| rs4149945 | 240097056 | 0.99 | 0.53 | 0.53 |
| rs2797599 | 240099067 | 0.99 | 0.93 | 0.59 |
| rs4149952 | 240099314 | 0.98 | 0.42 | 0.52 |
| rs4149963 | 240102005 | 0.91 | 0.75 | 0.86 |
| rs4149965 | 240102061 | 0.72 | 0.03 | 0.05 |
| rs17391703 | 240107314 | 0.73 | 0.04 | 0.09 |
| rs1047840 | 240108924 | 0.62 | 0.81 | 0.92 |
| rs1776148 | 240109168 | 0.65 | 0.42 | 0.63 |
| rs1418761 | 240109509 | 0.98 | 0.08 | 0.21 |
| rs1776149 | 240110494 | 0.98 | 0.05 | 0.17 |
| rs9350 | 240115297 | 0.84 | 0.25 | 0.06 |
| rs4408133 | 240116272 | 0.66 | 0.51 | 0.41 |
| rs4150018 | 240119244 | 0.55 | 0.18 | 0.27 |
| rs4150024 | 240119777 | 0.96 | 0.56 | 0.55 |

| | | | | |
|---|---|---|---|---|
| * Novel non-synonymous SNP [A/T] in exon 8 leading to an exchange of Asp for Val (according to Ensembl Genome Browser at http://www.ensembl.org). ** *P*_{CCG} cannot be computed because of zero homozygotes for the minor allele. major AF: major allele frequency in controls. *P*_{CCA}: *P* value obtained from an allele-based case-control χ² test. *P*_{CCG}*: P* value obtained from a genotype-based case-control χ² test. The *P* values are derived from the German sample for all genotyped SNPs at the *EXO1* locus (tagging SNPs, cSNPs, SNPs located in the promoter and 5' UTR regions). | | | | |

**Table 6: Fine mapping of the TCF3 gene region**

| **dbSNP ID** | **position build 36** | **major AF** | ***P*_{CCA}** | ***P*_{CCG}** |
|---|---|---|---|---|
| rs887390 | 1551035 | 0.97 | 0.56 | 0.60 |
| rs1052918 | 1560669 | 0.81 | 0.91 | 0.82 |
| rs3746112 | 1561556 | 1 | * | * |
| rs1052774 | 1566417 | 1 | * | * |
| rs917358 | 1568822 | 0.99 | 0.17 | ** |
| rs8140 | 1570339 | 0.60 | 0.84 | 0.29 |
| rs11879402 | 1573372 | 1 | * | * |
| rs740435 | 1584923 | 0.98 | 0.57 | 0.56 |
| rs10409667 | 1589919 | 0.81 | 0.32 | 0.60 |
| rs4807942 | 1593507 | 0.84 | 0.40 | 0.57 |
| rs1860661 | 1601134 | 0.60 | 0.90 | 0.94 |

| | | | | |
|---|---|---|---|---|
| * Monomorphic ** *P*_{CCG} cannot be computed because of zero homozygotes for the minor allele major AF: major allele frequency in controls. *P*_{CCA}: *P* value obtained from an allele-based case-control χ² test. *P*_{CCG}: *P* value obtained from a genotype-based case-control χ² test. The *P* values were obtained from the German sample for all SNPs genotyped at the *TCF3* locus (tagging SNPs, cSNPs, SNPs located in the promoter and 5' UTR regions). | | | | |

**Table 7: Primer sequences used for the mutation detection of EXO1**

| **region** | **primer** | **sequence** | **amplicon** |
|---|---|---|---|
| promoter | EXO1_p_F EXO1_p_R | CTAAAGTCTTTGTCTCTAAGGCCCA CTTCCAACTCATAGGGTTGCG | 551 bp |
| exon 01 | EXO1_p_e01_F EXO1_e01_R | CATCAGCGCCAGTGCCAC AACACATACTCCACTCAGGCTAAGG | 551 bp |
| exon 02 | EXO1_e02_F EXO1_e02_R | TTCAGTGAACGGAGGGAGATAAGAG GTTCTCAAATTTCAGAGGTTCTTAGACTC | 609 bp |
| exon 02a | EXO1_e02a_F EXO1_e02a_R | TGCCTGGCCCAGAAGAAAC AGTCAGTAATGGGCACTGAGCA | 401 bp |
| exon 03 | EX01_e03_F EXO1_e03_R | ATTGGACTCCAAGCTTTCTCTTCA GGAAGTCATCCCTGATTTGGG | 433 bp |
| exon 04 | EXO1_e04_F EXO1_e04_R | CAATTCCTATGAAAACATCTGCTT TTAAAAATTGGTATCATATAGACTACAGTGTC | 338 bp |
| exon 05 | EXO1_e05_F EXO1_e05_R | AAGCCTAAAGCATCTGGGTTAATG GAGAGAGCCTTACTTATGCCTTAGGA | 502 bp |
| exon 06 | EXO1_e06_F EXO1_e06_R | TCCTCTGGCTGTGTAGCAGTATTTC CATTACACAAACATCAAGCAGCAA | 640 bp |
| exon 07 | EXO1_e07_F EXO1_e07_R | ATAAAGAGATGCAAAAAGAGCTTGC AGTTCTAGTGCCTCAGTCATTTGCT | 618 bp |
| exon 08 | EXO1_e08_F EXO1_e08_R | GAATTTTAAGTGATGTAACATGGTTGTG CTAAGTAATCAGGATGCATAGTGGATG | 582 bp |
| exon 09 | EX01_e09_F EXO1_e09_R | AAGACACCAACTCCTGCAGAGAC CAGAGCCTGGATATAGAAACGCTTA | 414 bp |
| exon 10 | EXO1_e10_F EXO1_e10_R | ACAGCTGTTTCATCAACAGCTATTG GGTATGTGTTTATCAGCAGGGTGA | 511 bp |
| exon 11 | EXO1_e11_F EXO1_e11_R | AAGTCCTTATTTTTAGGACAATGGGA TATACCCATGTAACAAAACTGCACG | 496 bp |
| exon 12 | EXO1_e12_1_F EXO1_e12_1_R EXO1_e12_2_F EXO1_e12_2_R | AATTCTAAATGTGTGAATGTTGGCA GCGTTCTTGAAAAATCTCCAAGAC TGGTTGACACAGATGTAGCACG TGTGAACTTTCTGCACAGTAGAGTTTAG | 551 bp 606 bp |
| exon 13 | EXO1_e13_F EXO1_e13_R | CTTCTTCAATGAAATTGGCAAATATC ACACTGCAAGAGAAAATAAGTTTCACA | 461 bp |
| exon 14 | EXO1_e14_F EXO1_e14_R | GCCGAGATCGCACCACTG AACAAAAACTCATACGAAACTAGGAAGA | 457 bp |
| exon 15 | EXO1_e15_F EXO1_e15 R | ATTGGGAGTGCTCACAGGAGAAC AACTCCAGAGGATTTTCCCAATTA | 576 bp |

The exon - numbering follows the NCBI nomenclature for NM_003686. The additional exon of the NM_130398 isoform is here referred to as 02a. The nucleotide sequences given in table 7 are equal to SEQ ID NO: 3 to 38.

### REFERENCES

1. Vijg, J. Somatic mutations and aging: a re-evaluation. Mutat. Res. 447, 117-135 (2000).
2. Hasty, P., Campisi, J., Hoeijmakers, J., van Steeg, H., Vijg, J. Aging and genome maintenance: lessons from the mouse? Science 299, 1355-1359 (2003).
3. Hasty, P. The impact of DNA damage, genetic mutation and cellular responses on cancer prevention, longevity and aging: observations in humans and mice. Mech. Ageing Dev. 126, 71-77 (2005).
4. Lombard, D.B. et al. DNA repair, genome stability, and aging. Cell 120, 497-512 (2005).
5. Hart, R.W., Setlow, R.B. Correlation between deoxyribonucleic acid excision-repair and life-span in a number of mammalian species. Proc. Natl. Acad. Sci. USA 71, 2169-2173 (1974).
6. Kirkwood, T.B. Understanding the odd science of aging. Cell 120, 437-447 (2005).
7. Hitt, R., Young-Xu, Y., Silver, M., Perls, T. Centenarians: the older you get, the healthier you have been. Lancet 354, 652 (1999).
8. Perls, T., Kunkel, L.M., Puca, A.A. The genetics of exceptional human longevity. J. Am. Geriatr. Soc. 50, 359-368 (2002).
9. Hjelmborg, J. et al. Genetic influence on human lifespan and longevity. Hum. Genet. 119, 312-321 (2006).
10. Christensen, K., Johnson, T.E., Vaupel, J.W. The quest for genetic determinants of human longevity: challenges and insights. Nat. Rev. Genet. 7, 436-448 (2006).
11. Franceschi, C. et al. Do men and women follow different trajectories to reach extreme longevity? Italian Multicenter Study on Centenarians (IMUSCE). Aging (Milano) 12, 77-84 (2000).
12. Candore, G. et al. Immunogenetics, gender, and longevity. Ann. N. Y Acad. Sci. 1089, 516-537 (2006).
13. Qiu, J., Qian, Y., Chen, V., Guan, M.X., Shen, B.Human exonuclease 1 functionally complements its yeast homologues in DNA recombination, RNA primer removal, and mutation avoidance. J. Biol. Chem. 274, 17893-17900 (1999).
14. Liberti, S.E., Rasmussen, L.J. Is hEXO1 a cancer predisposing gene? Mol. Cancer Res. 2, 427-432 (2004).
15. Sharma, S. The exonucleolytic and endonucleolytic cleavage activities of human exonuclease 1 are stimulated by an interaction with the carboxyl-terminal region of the Werner syndrome protein. J. Biol. Chem. 278, 23487-23496 (2003).
16. Maringele, L., Lydall, D. EXO1-dependent single-stranded DNA at telomeres activates subsets of DNA damage and spindle checkpoint pathways in budding yeast yku70Delta mutants. Genes Dev. 16, 1919-1933 (2002).
17. Schaetzlein, S. et al. Exonuclease-1 deletion impairs DNA damage signaling and prolongs lifespan of telomere-dysfunctional mice. Cell 130, 863-877 (2007).
18. Wei, K. Inactivation of exonuclease 1 in mice results in DNA mismatch repair defects, increased cancer susceptibility, and male and female sterility. Genes Dev. 17, 603-614 (2003).
19. Slattery, C., Ryan, M.P., McMorrow, T. E2A proteins: Regulators of cell phenotype in normal physiology and disease. Int. J. Biochem. Cell Biol. Epub ahead of print (2007).
20. Schwartz R, Engel I, Fallahi-Sichani M, Petrie HT, Murre C. Gene expression patterns define novel roles for E47 in cell cycle progression, cytokine-mediated signaling, and T lineage development. Proc Natl Acad Sci U S A. 2006 Jun 27;103(26):9976-81
21. Frasca D, Nguyen D, Riley RL, Blomberg BB. Decreased E12 and/or E47 transcription factor activity in the bone marrow as well as in the spleen of aged mice. J Immunol. 2003 Jan 15;170(2):719-26.
22. Frasca D, Nguyen D, Riley RL, Blomberg BB. Effects of aging on proliferation and E47 transcription factor activity induced by different stimuli in murine splenic B cells. Mech Ageing Dev. 2003 Apr;124(4):361-9.
23. Zheng W, Wang H, Xue L, Zhang Z, Tong T. Regulation of cellular senescence and p16(INK4a) expression by Id1 and E47 proteins in human diploid fibroblast.J Biol Chem. 2004 Jul 23;279(30):31524-32.
24. Murre, C. Helix-loop-helix proteins and lymphocyte development. Nat. Immunol. 6, 1079-1086 (2005).
25. Perls T, Terry D. Understanding the determinants of exceptional longevity. Ann. Intern. Med. 139,445-449 (2003).
26. Nebel, A. et al. No association between microsomal triglyceride transfer protein (MTP) haplotype and longevity in humans. Proc. Natl. Acad. Sci. USA 102, 7906-7909 (2005).
27. Steffens, M. et al. SNP-based analysis of genetic substructure in the German population. Hum. Hered. 62, 20-29 (2006).
28. Blanché, H., Cabanne, L., Sahbatou, M., Thomas, G. A study of French centenarians: are ACE and APOE associated with longevity? Comptes Rendus de l'Académie des Sciences 324, 129-135 (2001).
29. Hampe, J. et al. A genome-wide association scan of nonsynonymous SNPs identifies a susceptibility variant for Crohn disease in ATG16L1. Nat. Genet. 39, 207-211 (2007).
30. Weckx, S. et al. novoSNP, a novel computational tool for sequence variation discovery. Genome Res. 15, 436-442 (2005).
31. Till, A. et al. The Met-196 -> Arg variation of human tumor necrosis factor receptor 2 (TNFR2) affects TNF-alpha-induced apoptosis by impaired NF-kappaB signaling and target gene expression. J. Biol. Chem. 280, 5994-6004 (2005).
32. Arlt, A. et al. The expression of immediate early gene X-1 (IEX-1) is differentially induced by retinoic acids in NB4 and KG1 cells: possible implication in the distinct phenotype of retinoic acid-responsive and -resistant leukemic cells. Leukemia 18, 1646-1655 (2004).
33. Krawczak, M. et al. PopGen: population-based recruitment of patients and controls for the analysis of complex genotype-phenotype relationships. Community Genet. 9, 55-61 (2006).
34. Livak, K.J., Schmittgen, T.D. Analysis of relative gene expression data using real-time quantitative PCR and the 2(-Delta Delta C(T)) method. Methods 25, 402-408 (2001).

## Claims

1. A method of identifying individuals having a nucleic acid molecule not associated with exceptional life expectancy, comprising the steps of
(a) amplifying a DNA fragment comprising an individual's nucleotide sequence selected from the group of:
(i) nucleic acid molecules comprising the nucleotide sequence as depicted in SEQ ID NO: 2,
(ii) nucleic acid molecules the complementary strand of which hybridizes to a nucleic acid molecule of (i) and
(iii) nucleic acid molecules the sequence of which differs from the sequence of a nucleic acid molecule of (ii) due to the degeneracy of the genetic code,
using an oligonucleotide primer which specifically hybridizes to sequences within the individual's nucleotide sequence;
(b) sequencing said amplified DNA fragment;
(c) comparing the individual's nucleotide sequence with the nucleotide sequence as depicted in SEQ ID NO: 2;
(d) determining the absence of a polymorphic variation in the individual's nucleotide sequence at position 21 of the nucleotide sequence as depicted in SEQ ID NO: 2.

2. A method of identifying individuals having a nucleic acid molecule associated with exceptional life expectancy resulting from the presence of a polymorphism at position 21 in SNP rs1776180 of the EXO1 gene as depicted in SEQ ID NO: 1, comprising the steps of
(a) amplifying a DNA fragment comprising an individual's nucleotide sequence selected from the group of:
(i) nucleic acid molecules comprising the nucleotide sequence as depicted in SEQ ID NO: 1,
(ii) nucleic acid molecules the complementary strand of which hybridizes to a nucleic acid molecule of (i) and
(iii) nucleic acid molecules the sequence of which differs from the sequence of a nucleic acid molecule of (ii) due to the degeneracy of the genetic code,
using an oligonucleotide primers which specifically hybridizes to sequences within the individual's nucleotide sequence;
(b) sequencing said amplified DNA fragment;
(c) comparing the individual's nucleotide sequence with the nucleotide sequence as depicted in SEQ ID NO: 2;
(d) determining the presence of a polymorphic variation in the individual's nucleotide sequence at position 21 of the nucleotide sequence as depicted in SEQ ID NO: 2, wherein said polymorphic variation is a single nucleotide polymorphism, in which G is substituted by C.

3. Use of a vector comprising a nucleotide sequence of SEQ ID NO: 1 for preparing a pharmaceutical composition for the treatment of infectious and/or malignant diseases and/or diseases related to aging.

4. Use of a vector comprising a nucleotide sequence of SEQ ID NO: 1 for preparing a pharmaceutical composition for germline protection and stability.

5. Use of transcription factor E47 for the screening of substances affecting the expression of the EXO1 gene.

6. Use of transcription factor E47 according to claim 5, **characterized in that** E47 is used as a competing agent for the binding of a substance to a nucleotide sequence selected from the group of:
(i) the nucleotide sequence as depicted in SEQ ID NO: 2,
(ii) nucleic acid molecules the complementary strand of which hybridizes to a nucleic acid molecule of (i) and
(iii) nucleic acid molecules the sequence of which differs from the sequence of a nucleic acid molecule of (ii) due to the degeneracy of the genetic code.

7. Use of transcription factor E47 according to claim 5, **characterized in that** E47 is used as a target for the screening of substances affecting the expression of the EXO1 gene.

8. Use of a nucleotide sequence as depicted in SEQ ID NO: 39, 40, 41 and/or 42 for preparing a pharmaceutical composition for the treatment of infectious and/or malignant diseases and/or diseases related to aging.

9. Use of a nucleotide sequence as depicted in SEQ ID NO: 39, 40, 41 and/or 42 for preparing a pharmaceutical composition for germline protection and stability.
